# EUROPÄISCHE PATENTANMELDUNG

(11) **EP 2 071 947 A1**
(43) Veröffentlichungstag der Anmeldung: **24.06.2009**
(21) Anmeldenummer: 08011233.7
(22) Anmeldetag: 20.06.2008
(51) Int. Cl.: A01M 1/20, A61L 9/12, A61L 9/012, A61L 9/04

(54) **Vorrichtung und Verfahren zum Behandeln von Umgebungsluft**

(30) Priorität: 21.12.2007 DE 202007017927 U
(71) Anmelder: Air & D- Sarl, 67190 Rosheim (FR)
(72) Erfinder: Wuest Robert, 67190 Rosheim (FR); Vasko Miroslav, 67650 Grendelbruch (FR)
(74) Vertreter: Wagner, Jutta

(57) **Zusammenfassung**

Die vorliegende Erfindung stellt eine Vorrichtung zum Behandeln von Umgebungsluft mit Aktivstoffen bereit. Dabei umfasst die Vorrichtung ein Gehäuse mit einem ersten und einem zweiten Abschnitt (1,1'), wobei innerhalb des ersten Abschnitts Aktivstoffträgerelemente (2) angeordnet sind, und eine Vorrichtung zum Befördern von Luft (3) von außerhalb des Gehäuses in den ersten Abschnitt (1) des Gehäuses angeordnet ist. Die in das Gehäuse beförderte Luft passiert die Aktivstoffträgerelemente (2) passiert, und wird dann einem innerhalb des zweiten Abschnitts des Gehäuses (1') angeordneten Kompressor (5) zugeführt wird. Von diesem wird die Luft über einen Auslass (14) aus dem Gehäuse emittiert. Der Kompressor (5) ist mit der Vorrichtung zum Befördern von Luft (3) von außerhalb des Gehäuses in den ersten Abschnitt (1) des Gehäuses mittels einer Steuerungs- oder einer Steuerungs-Regelungsvorrichtung (12) operativ derart gekoppelt, dass die in das Gehäuse beförderte Luft-Masse (ΔM₁) innerhalb der Zeitspanne ΔT, die einer Anzahl n von Zyklen des Kompressors entspricht, gleich der Luft-masse (ΔM₂) ist, die durch den Auslass (14) innerhalb dieser Zeitspanne ΔT emittiert wird.

## Beschreibung

Die Erfindung betrifft eine Vorrichtung zum Behandeln von Umgebungsluft mit Aktivstoffen.

### STAND DER TECHNIK

Vorrichtungen zum Beduften von Räumen oder zur Reinhaltung der Luft sind aus dem Stand der Technik bereits bekannt. So beschreibt die EPA 1 593 397 eine Vorrichtung, die eine Säule umfasst, die mit einem gelförmigen Trägermaterial, an dem flüssige Desodorantien adsorbiert sind, beschickt ist. Am Boden der Säule ist ein Ventilator angebracht, durch den Umgebungsluft angesaugt wird. Diese strömt an dem Trägermaterial vorbei und setzt die Desodorantien frei. Die mit den Desodorantien beladene Luft tritt durch eine Vielzahl von seitlich an der Säule angebrachte Öffnungen in den umgebenden Raum aus.

Weiter beschreibt das Gebrauchsmuster DE 20 2007 017 927 U1 eine Vorrichtung, in der in einem Kasten Einsätze mit Gelträgerplatten angeordnet sind, an denen aktive Agenzien adsorbiert sind und wobei in dem Kasten ein Ventilator und ein Kompressor angeordnet sind, so dass Luft in den Kasten hinein befördert und unter Druck nach Beladung mit aktiven Agenzien wieder hinausbefördert werden kann.

Die in den Vorrichtungen des Standes der Technik werden die aktiven Agenzien auf inhomogene Weise abgebaut, so dass die Verbesserung der Luftqualität nicht zuverlässig gleich bleibend ist.

Insofern besteht das Erfordernis, eine verbesserte Vorrichtung zum Behandeln von Umgebungsluft mit Aktivstoffen zu schaffen, die eine homogenere Behandlungsqualität ermöglicht.

### OFFENBARUNG

Ausgehend von diesem Stand der Technik liegt der vorliegenden Erfindung die Aufgabe zu Grunde, eine verbesserte Vorrichtung und ein Verfahren zum Behandeln von Umgebungsluft mit Aktivstoffen zu schaffen. Diese Aufgabe wird durch eine Vorrichtung mit den Merkmalen des unabhängigen Anspruchs 1 und durch ein Verfahren mit den Merkmalen des unabhängigen Anspruchs 11 gelöst. Weiterbildungen des Gegenstands sind in den entsprechenden Unteransprüchen offenbart.

Eine erste Ausführungsform der erfindungsgemäßen Vorrichtung bezieht sich auf eine Vorrichtung zum Behandeln von Umgebungsluft mit Aktivstoffen. Die Vorrichtung besteht aus einem Gehäuse, das in zwei Abschnitte unterteilt ist und in dessen einem Abschnitt Aktivstoffträgerelemente angeordnet sind, die von Umgebungsluft umströmt werden, die mittels einer geeigneten Vorrichtung zum Befördern von Luft von außerhalb des Gehäuses in den ersten Abschnitt des Gehäuses zugeführt wird. Die Luft reichert sich beim Umströmen der Aktivstoffträgerelemente mit entsprechenden Aktivstoffen an, wird sodann in den zweiten Abschnitt des Gehäuses überführt, wo sie mit Hilfe eines Kompressors komprimiert wird, so dass sie vorteilhaft unter Druck aus der Vorrichtung an einen gewünschten Ort entlassen werden kann. Vorteilhaft sind die beiden Vorrichtungen zum Befördern der Luft in den ersten Abschnitt und zum Komprimieren der Luft mittels einer Regelungs- oder Steuer- und Regelungsvorrichtung derart miteinander gekoppelt, dass sie voneinander abhängig agieren. Dabei wird vorteilhaft ermöglicht, dass die während einer bestimmten Zeitdauer in das Gehäuse beförderte Luft-Masse gleich groß ist wie die aus dem Gehäuse in derselben Zeit emittierte Luftmasse. Damit wird vorteilhaft erreicht, dass kein Über- oder Unterdruck in dem Gehäuse entsteht und dass ein gleichmäßiger Aktivstoffabbau in den Aktivstoffträgerelementen erreicht wird.

Weitere Ausführungsformen der Vorrichtung beziehen sich darauf, dass entweder die Antriebsmotoren des Kompressors und der Vorrichtung zum Fördern von Luft in das Gehäuse synchronisiert sind, was ebenfalls über die Steuerungs- oder der Steuerungs-Regelungsvorrichtung, kurz, des Reglers, erreicht wird, oder dass die operative Kopplung der beiden Geräte über Vorrichtungen wie Zeitgeber oder Taktgeber geschaffen wird. Damit kann vorteilhaft ermöglicht werden, dass in Zeiten, in denen ein höherer Umgebungsluftdurchsatz erforderlich ist, um die gewünschte Luftverbesserungsqualität zu erzielen, die Leistung der Geräte zusammen gleichzeitig oder synchron hochgefahren wird.

Weitere Ausführungsformen beziehen sich darauf, dass die Änderung der Betriebsleistung des Kompressors und der Vorrichtung zum Fördern von Luft kontinuierlich oder stufenweise veränderbar ist.

Schließlich stellen weitere Ausführungsformen beispielhaft dar, dass die Betätigung des Reglers bequem mittels eines Bedienfeldes, das an der Außenseite des Gehäuses angeordnet ist, vorgenommen werden kann.

Eine noch weiteres Ausführungsform legt dar, dass besonders vorteilhaft auch eine Kommunikation des Reglers zu einem Sensor die Steuerung der beiden Vorrichtungen Kompressor und Vorrichtung zum Befördern von Luft bereitstellen kann, wobei es besonders vorteilhaft ist, dass bei bestimmten, von dem Sensor gemessenen Umgebungsbedingungen diese Geräte mit höherer Leistung arbeiten oder gar abgeschaltet werden.

Schließlich beziehen sich Ausführungsformen darauf, dass Zwischenbehälter als Speichersysteme zwischen Kompressor und Verbrauchersystem vorgesehen sein können, so dass unabhängig von der Verbrauchszeit oder bei Bedarf einer größeren Menge an mit Aktivstoff beladener Luft diese bereit gestellt werden kann.

Diese und weitere Vorteile werden aus der nachfolgenden Beschreibung und den begleitenden Figuren ersichtlich.

### KURZBESCHREIBUNG DER FIGUREN

Der Bezug auf die Figuren in der Beschreibung dient der Unterstützung der Beschreibung. Gegenstände oder Teile von Gegenständen, die im Wesentlichen gleich oder ähnlich sind, können mit denselben Bezugszeichen versehen sein. Die Figuren sind lediglich eine schematische Darstellung einer Ausführungsform der Erfindung.

**Fig. 1** zeigt eine perspektivische Vorderansicht der erfindungsgemäßen Vorrichtung,

**Fig. 2** zeigt eine graphische Darstellung der Messung der Emission von flüchtigen aktiven Substanzen mittels der erfindungsgemäßen Vorrichtung über einen bestimmten Zeitraum.

### BESCHREIBUNG

Unter Umgebungsluft, die mittels der vorliegenden Erfindung behandelt werden soll, wird vorliegend Luft jedwederArt verstanden, die aus geruchlichen oder gesundheitlichen oder anderen Gründen hinsichtlich ihrer Qualität verbessert werden soll. Die Qualitätsverbesserung kann dabei eine Verbesserung der in der Luft wahrnehmbaren Gerüche sein, indem einer übel riechenden Luft, etwa aus einem Toilettenraum oder einer Bratküche, Düfte zugefügt werden, die diese üblen Gerüche überlagern, etwa Zitronenduft oder andere Düfte etherischer Öle, wobei die Düfte aus natürlichen oder künstlichen Duftquellen stammen können. Die Qualitätsverbesserung kann auch eine tatsächliche Veränderung von Substanzen sein, die den Geruch der Luft nachteilig beeinträchtigen und die beispielsweise chemisch durch Abbau oder Modifikation verändert werden oder die physikalisch, beispielsweise durch Einschluss in Käfigmoleküle, unschädlich gemacht werden.

Unter aktiven Substanzen oder Aktivsubstanzen werden folglich die Substanzen verstanden, die geeignet sind, aktiv eine Änderung der Luftqualität herbeizuführen. Es können dies Aldehyde, Terpene, Ester sein, Insektizide, Biozide, Desodorantien und weitere Stoffe, wie sie bereits aus der EP-A 1 334 735 bekannt sind.

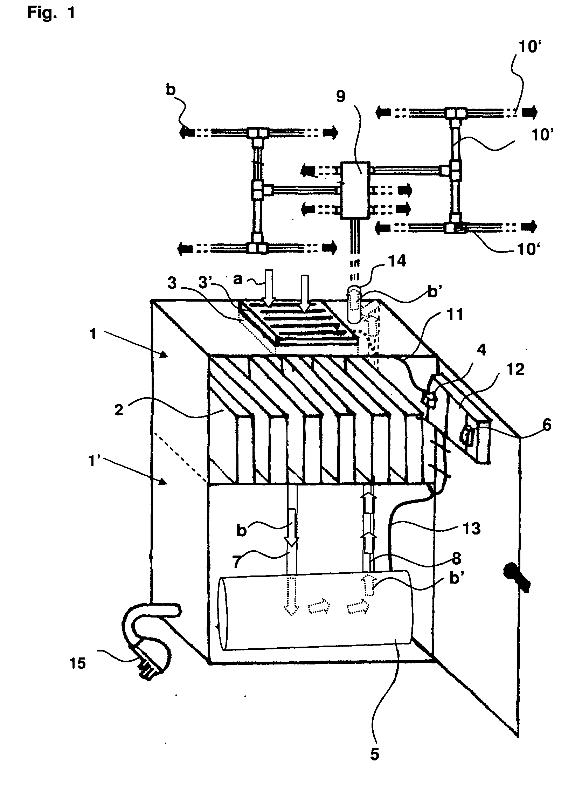

Allgemein formuliert, handelt es sich bei der Vorrichtung zum Behandeln von Umgebungsluft mit Aktivstoffen um ein Gehäuse, das in einen ersten Abschnitt und einen zweiten Abschnitt unterteilt ist. In dem ersten Abschnitt sind Aktivstoffträgerelemente angeordnet, bei denen es sich vorteilhaft um Kassetten handelt, die mit einem Trägermaterial für Aktivstoffe gefüllt sind. Die Kassetten sind dabei für die Aktivstoffe, die in einem Temperaturbereich ab 15 °C aus dem Trägermaterial in die Umgebungsatmosphäre übertreten, durchlässig, so dass das Material verbraucht wird. Besonders vorteilhaft sind Kassetten des Typs AirForce ®, erhältlich bei Fa. Biothys GmbH, Wilstätt, Deutschland, die wegen ihrer plattenförmigen Geometrie in der erfindungsgemäßen Vorrichtung geschickt angeordnet werden können. Zweckmäßig ist der erste Abschnitt des Gehäuses so gestaltet, dass er einen Boden aufweist, auf dem eine Vielzahl von Kassetten senkrecht stehend angeordnet werden kann, wie **Fig. 1** zeigt:

Dort ist der erste Abschnitt zugleich ein oberer Abschnitt 1, der über einem unteren Abschnitt 1' angeordnet ist. Das gezeigte Gehäuse kann stehend angeordnet werden; an seiner oberen Seite ist ein Vorrichtung zum Befördern von Luft 3 von außerhalb des Gehäuses in den ersten Abschnitt 1 bereitgestellt, vorliegend angedeutet durch das strichelierte Gehäuse 3 unterhalb des Abdeckgitters 3'. Dieses deckt die notwendige Öffnung an der Gehäuseoberseite nach oben ab und schützt die eigentliche Vorrichtung 3, die Luft in das Gehäuse zuführt und die in jedem Fall eine Pumpfunktion ausüben muss. Es kann sich hierbei um einen entsprechend betriebenen Ventilator, oder auch um eine geeignete andere Pumpe wie eine Flügelradpumpe oder eine Flügelzellenpumpe handeln. Dem Fachmann sind weitere Vorrichtungen zum Ansaugen von Luft und Überführen in ein Gehäuse bekannt.

Die Luftzufuhr in das Gehäuse kann also über die Leistung des Ventilators oder der entsprechenden Vorrichtung geregelt werden; ein Dimmer kann zu diesem Zweck vorgesehen sein. Anpassung der Luftuzufuhr verhindert, dass ein Überdruck im Gehäuse entsteht und dass nur soviel Luft in das Gehäuse gefördert wird, wie die Kassetten benötigen, um eine maximale Aktivstoffabgabe zu leisten. Entsprechend werden die Luftzufuhr und der Kompressor aneinander angepasst.

Die in **Fig. 1** gezeigte Anordnung stellt eine Vielzahl senkrecht nebeneinander angeordneter Kassetten, respektive plattenförmiger Aktivstoffträgerelemente dar, die senkrecht stehend nebeneinander angeordnet sind, wobei die Platten vorteilhaft mit einem Zwischenraum von zumindest 5 mm voneinander beabstandet sind, so dass die von oben einströmende Luft die Platten gut umströmen kann. Wird ein Ventilator oder eine andere Vorrichtung zur Erzeugung turbulenter Strömung gewählt, so ist dies besonders vorteilhaft, da dann ein möglichst gleichmäßiges Passieren der Luft an allen Aktivstoffträgerelementen 2 ermöglicht wird und somit die Aktivstoffe aus allen Platten gleichmäßig ausgetragen und an die passierende Luft abgegeben werden.

Die mit den Aktivstoffen beladene Luft wird nunmehr in den zweiten, in **Fig. 1** unteren Abschnitt 1' und dort in den Kompressor 5 überführt. Vorliegend wird dies mittels des gezeigten Überführungsrohrs 7 realisiert. Selbstverständlich können mehrere oder anders angeordnete Überführungsmöglichkeiten gewählt werden, abhängig vom Typ des Kompressors und von dem zur Verfügung stehenden Raum. Dann wird die mit Aktivstoffen beladene Luft komprimiert und unter Druck stehend über das Abführrohr 8 in einen Auslass 14 aus dem Gehäuse emittiert wird. Für das Abführrohr 8 und den Auslass 14 gilt das für das Überführungsrohr 7 gesagte: Auch hier können unterschiedliche Anzahlen und Anordnungen gewählt werden, abhängig von dem Druck, mit dem die Luft emittiert werden soll, und vom Kompressortyp oder dem zur Verfügung stehenden Platz.

Der Kompressor 5 und die Vorrichtung zum Befördern von Luft 3 von außerhalb des Gehäuses in den ersten Abschnitt 1 des Gehäuses werden dabei mittels der Steuerungs-Regelungsvorrichtung 12 operativ derart gekoppelt, dass die in das Gehäuse beförderte Luft-Masse ΔM₁, angedeutet durch den Pfeil a, innerhalb der Zeitspanne ΔT, die einer Anzahl n von Zyklen des Kompressors entspricht, gleich der Luftmasse ΔM₂ ist, angedeutet durch den Pfeil b', die durch den Auslass 14 innerhalb dieser Zeitspanne ΔT emittiert wird. Damit wird sichergestellt, dass zum einen kein Überdruck im Gehäuse entsteht, und zum anderen wird eine gleichmäßige Überführung von Aktivstoffen in die aufnehmende Luft ermöglicht.

Wenn stets nur so viel Luft mit Aktivstoff beaufschlagt wird, wie von dem System, das die mit Aktivstoff beladene Luft aufnimmt, gefordert wird, so kann ökonomischer, homogener Verbrauch des Aktivstoffs aus den Kassetten ermöglicht werden, was gleichzeitig zu einer kontrolliert guten Luftqualität der behandelten Luft führt, also der Luft, die mit der behandelten Luft angereichert wird, sozusagen das Empfängersystem.

Um die Vorgabe zu erreichen, dass während einer bestimmten Zeitspanne ΔT, in der der Kompressor eine Vielzahl von Kompressions- und Entspannungsvorgängen ausführt, die zusammen jeweils einen Zyklus darstellen, die gleiche Masse an Luft in das Gehäuse eingeführt, wie aus ihm entlassen wird, können etwa die Antriebsmotoren des Kompressors und der Vorrichtung zum Befördern von Luft, etwa dem Ventilator, synchronisiert werden, wenn ihre Leistungen bekannt und aufeinander abgestimmt sind. Der Kompressor, mittels dessen Hilfe die Beförderung und die Verteilung der Aktivstoffluft getätigt wird, kann auch genutzt werden um durch seine Abwärme die Temperatur im Gehäuse auf eine Temperatur zu bringen, bei der der Aktivstoff ideal in die Luft übertritt.

Weiter ist es möglich, die hinsichtlich ihrer Leistung aufeinander abgestimmten Geräte - Kompressor und Ventilator oder Pumpe - mittels der Steuerungs- oder der Steuerungs-Regelungsvorrichtung mittels einer Zeitgebervorrichtung wie einer Zeitschaltuhr 6, gezeigt in **Fig. 1****,** oder alternativ einer Taktgebervorrichtung, gesteuert werden. Damit kann eingestellt werden, dass die erfindungsgemäße Vorrichtung im bekannten Umfeld so betrieben wird, dass in Stoßzeiten, in denen eine große Masse an schlechter Luft mit Aktivstoff beladener Luft behandelt werden soll, ein größerer Durchsatz der erfindungsgemäßen Vorrichtung eingestellt wird.

Die Änderung des Durchsatzes, die über eine Veränderung der Leistung einer der beiden Vorrichtungen Kompressor oder Vorrichtung zum Befördern von Luft erfolgen kann, kann stufenweise oder kontinuierlich erfolgen. Die Leistungsänderung kann über einen Drehregler, einen Schalter oder über ein entsprechendes Bedienfeld erfolgen. Derartige Anordnungen sind dem Fachmann bekannt. Vorteilhaft zeigt eine Anzeige die jeweilige Leistung an, mit der die Vorrichtung zum Befördern von Luft und mit der der Kompressor betrieben wird Eine Änderung der Leistung des einen Geräts bewirkt durch die operative Kopplung mit dem jeweils anderen Gerät, dass dessen Leistung entsprechend angepasst wird.

Überdies kann die Steuerungs-Regelungsvorrichtung der erfindungsgemäßen Vorrichtung mit einem Sensor operativ gekoppelt sein, der Veränderungen in der Umgebungsluft wie Rauch, eine über den gewünschten Temperatur- und Betriebsbereich der Vorrichtung hinausgehende Temperatur, etwa eine Außentemperatur von über 40 °C oder von unter 10 °C oder sogar noch darunter, etwa um den Gefrierpunkt, oder eine über ein gewünschtes Maß hinausgehende Luftfeuchtigkeit oder sogar das Vorhandensein schädigender Chemikalien in der Luft detektiert und bei entsprechender Signalübermittlung an den Regler bewirkt, dass die Leistung der erfindungsgemäßen Vorrichtung den veränderten Bedingungen angepasst wird. Damit kann beispielsweise ein Selbstausschalten der Vorrichtung bei zu hohen Außentemperaturen bewirkt werden, um nicht unerwünscht Aktivstoff aus dem Trägermaterial in zu großer Menge auszutragen.

Die Übertragung der Signale von dem Sensor zu der Steuerungs- und Regelungsvorrichtung oder auch von dem Kompressor oder der Vorrichtung zur Beförderung von Luft in das Gehäuse kann auf konventionelle Weise geschehen. So zeigt **Fig. 1** elektronische Kabelverbindungen 11,13 zwischen dem Kompressor 5 und Regler 12 bzw. zwischen der Vorrichtung zur Beförderung von Luft 3 und dem Regler 12. Ein Stellschalter 4 dient dazu, den Regler ein- und auszuschalten. Über den Netzstecker 15 können Kompressor, Regler und Ventilator mit Energie versorgt werden.

Alternativ können statt der elektronischen Kabelverbindungen 11,13 auch Signalübertragungsmittel wie eine berührungslose elektromagnetische Signalübertragungsvorrichtung, insbesondere eine optische oder mittels Funkwellen bereitgestellte Signalübertragungsvorrichtung, eine kapazitive Signalübertragungsvorrichtung oder eine induktive Signalübertragungsvorrichtung zwischen dem Sensor und dem Regler oder entsprechend zwischen Kompressor oder der Vorrichtung zur Beförderung von Luft in das Gehäuse und dem Regler gewählt werden.

Vorliegend ist das Gehäuse, wie in **Fig. 1** gezeigt, ein Schrank, dessen Vorderseite als drehgelenkig angelenkte Tür ausgebildet ist, so dass vorteilhaft alle umfassten Vorrichtungen in dem Gehäuse geschickt gewartet werden können und die Kassetten 2, wenn der Aktivstoff verbraucht ist, ausgetauscht werden können.

Alternativ könnte die Öffnung eines Gehäuses, das geeignet ist zur Aufnahme der an der Erfindung beteiligten Komponenten, auch durch eine Schiebetür, eine Klappe oder eine Frontplatte eines Einschubelements verschlossen werden.

Vorteilhaft wird in einer Vorrichtung, wie sie in **Fig. 1** gezeigt ist, eine Zuführung der Luft, um die Kassetten zu umströmen, von der Oberseite des Gehäuses erfolgen; die Luftemission aus dem Kompressor 5 ist vorliegend auch über die Oberseite des Gehäuses mittels eines Auslasses 14 gezeigt, der in einen Verteiler 9 mündet, von dem aus sich über Verteilerrohrkomponenten 10 - wie T-Stücke - eine Vielzahl von Verteilerrohren 10' in die zu beschickenden Räume oder Bereiche, die auch außerhalb von Gebäuden liegen können, erstrecken. Wie gezeigt durch Pfeile b, strömt die entspannte, mit Aktivstoff beladene Luft durch das Verteilersystem.

Abhängig von der Art der Verteilung und dem zu beschickenden Raumvolumen wird die erfindungsgemäße Vorrichtung hinsichtlich der Kompressorleistung, der Leistung der Zuführvorrichtung für Luft und der Anzahl der plattenförmigen Aktivstoffträgerelemente gewählt. So können Kompressoren Leistungen im Bereich von 5 bis über 500 l/min aufweisen, was jedoch nicht beschränkend sondern lediglich beispielhaft angeführt wird. Der Fachmann weiß, wie ein Kompressor ausgelegt sein muss, um ein vorgegebenes Raumvolumen mit einer vorgegebenen Masse an Luft über einen bestimmten Zeitraum zu beschicken. Selbstverständlich können sehr große Anlagen über Kompressoren mit höherer Leistung verfügen.

Industrieanlagen können durchaus eine Vielzahl von einzelnen Gehäusen, die die erfindungsgemäße Vorrichtung enthalten, in Reihe geschaltet oder parallel geschaltet aufweisen und damit ein großes Verteilersystem speisen. Ebenso ist es auch möglich, eine Vielzahl von Kompressoren jeweils in einem zweiten Abschnitt eines Gehäuses zu vereinigen und eine entsprechende Anzahl von plattenförmigen Aktivstoffträgerelementen, respektive eine entsprechende Mehrzahl von Luftzuführvorrichtungen in einem ersten Gehäuse anzuordnen.

Eine weitere Möglichkeit zur Beschickung großer Verbrauchersysteme wie etwa einer Anlage aus der Petrochemie besteht darin, zwischen den Auslass aus dem Kompressor, respektive aus Gehäuse, dem in dem sich der Kompressor befindet, einen oder mehrere Zwischenbehälter zu schalten, in den oder in die die kontinuierlich produzierte, mit Aktivstoff beladene Luft zwischengespeichert wird. Der Behälter ist vorteilhaft ein Druckbehälter und hält Drücken im Bereich von Atmosphärendruck bis zumindest 2 bar, wenn nötig auch bis hin zu 10 bar oder bis zu 15 bar stand. Damit kann das unter Druck Stehende Luft-Aktivstoffgemisch bei Bedarf in das Verteilersystem überführt werden.

Die Bevorratung hat verschiedene Vorteile: so kann in Zeiten, wenn wenig Bedarf an mit Aktivstoff beladener Luft herrscht, die Vorrichtung zur Produktion derselben kontinuierlich weiterbetrieben werden und die Aktivstoffluft wird in dem Zwischenbehälter bevorratet bis sie abgerufen wird. Zum anderen kann das gesamte System in Bereichen eingesetzt werden, in denen Explosionsschutz herrschen muss und in dem aus diesem Grund keine Vorrichtungen vorliegen dürfen, die auf Grund von elektrischer Ladung durch Schalter oder andere Komponenten eine Explosion ausgelöst werden könnten. Die Zwischenbehälterlösung schafft die Möglichkeit, die Vorrichtung zur Beaufschlagung der Luft mit Aktivstoff entsprechend beabstandet von einem Explosionsschutzbereich zu positionieren. Durch den Druck-Zwischenbehälter wird eine Zuführung in das Verteilersystem ermöglicht, wobei lediglich ein Ventil des Verteilersystems, respektive das Ventil, das die Zuführleitung des Verteilersystems in die explosionsgeschützten Zone schließt, notwendig ist, um die Zuführung von Aktivstoffluft in den explosionsgeschützten Bereich zu überführen oder zu stoppen.

Zwischenbehälter können Größen im Bereich von 100 bis 1000 I aufweisen oder auch größer sein, je nach Bedarf. Besonders große Behälter ermöglichen, dass auch Systeme wie Mülldeponien, im Bedarfsfall mit Aktivstoffluft geflutet werden können.

Weiter ist es vorteilhaft, dass bei der Verwendung oder Zwischenschaltung mehrerer Zwischenbehälter der eine bereits wieder gefüllt werden kann, während aus dem anderen gerade das Verteilersystem geflutet wird oder aus dem anderen die Aktivstoffluft direkt an den Bestimmungsort übertritt, eventuell unter Verwendung lediglich eines Reduzierventils.

Die erfindungsgemäße Vorrichtung kann auch in einem Gehäuse untergebracht sein, in dem der erste und der zweite Abschnitt benachbart zueinander angeordnet sind. Dann können die Kassetten horizontal übereinander angeordnet werden, und auch die Luftzuführung wird vorteilhaft horizontal, also über eine Seite des ersten Abschnitts erfolgen. Der Kompressorauslass kann im zweiten oder im ersten Abschnitt angeordnet sein, abhängig davon, ob die emittierte, mit Aktivstoff beladene Luft unmittelbar an die Umgebung entlassen oder einem Verteilersystem zugeführt wird. Wenn sie unmittelbar in die Umgebung abgegeben wird, wird zweckmäßig der Ausgang an einer Seite der Vorrichtung liegen, die von der Seite, an der Luft in das Gehäuse zugeführt wird, abgewandt ist.

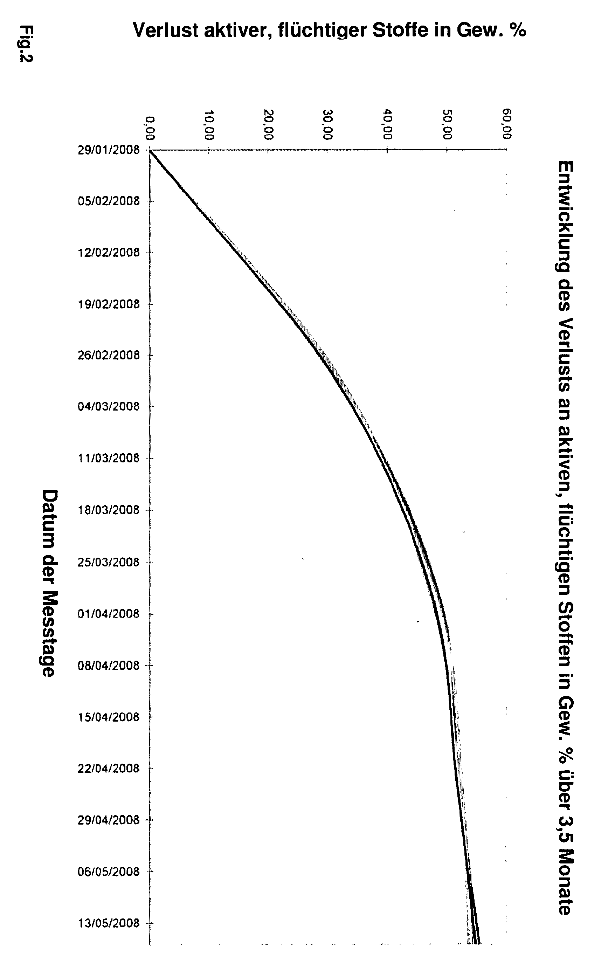

Fig. 2 zeigt eine graphische Darstellung der Messung der Emission von flüchtigen aktiven Substanzen mittels der erfindungsgemäßen Vorrichtung über einen bestimmten Zeitraum. Eine Anzahl von vier Kassetten des Typs AirForce 40/G wurde über den Zeitraum vom 29.01.2008 bis zum 13.05.2008 in einer Versuchsvorrichtung, die den erfindungsgemäßen Gegenstand darstellt, eingesetzt, und die Kassetten wurden an den angegebenen Tagen, siehe Datumsanzeige an der x-Achse der Graphik, gewogen, um den Verlust an Masse, die Trägersubstanz und Aktivstoff darstellt, zu erfassen. Es wird deutlich, dass der Abbau des Aktivstoffs kontinuierlich erfolgt und dass das Material keinem anfänglich starken Schwund unterliegt, sondern dass vielmehr ein nahezu linearer Abbau von Trägersubstanz und Aktivstoff stattfindet. Damit wird gezeigt, dass einen Zeitraum von dreieinhalb Monaten ein gleichmäßiges und damit optimiertes zur Verfügung Stellen an Aktivstoff erreicht wurde, bei stets gleich bleibender Einstellung der Betriebsweise. Idealerweise wird so der Aktivstoff optimal aus den Kassetten aufgebraucht.

### BEZUGSZEICHENLISTE

| | |
|---|---|
| 1 | Erster Abschnitt des Gehäuses |
| 1' | Zweiter Abschnitt des Gehäuses |
| 2 | plattenförmige Aktivstoffträgerelemente |
| 3 | Ventilator |
| 3' | Abdeckgitter des Ventilators |
| 4 | Stellschalter |
| 5 | Kompressor |
| 6 | Zeitschaltuhr |
| 7 | Zuführrohr für mit aktiven Substanzen beladene Umgebungsluft in den Kompressor |
| 8 | Abführrohr für komprimierte, mit aktiven Substanzen beladene Umgebungsluft aus dem Kompressor zu dem Auslass 14 |
| 9 | Verteiler |
| 10 | Verteilerrohrkomponente |
| 10' | Verteilerrohr |
| 11 | Elektr. Verbindung zw. Ventilator und Regler 12 |
| 12 | Regler |
| 13 | Elektr. Verbindung zw. Kompressor und Regler 12 |
| 14 | Auslass |
| 15 | Netzstecker |
| a | Umgebungsluftstrom |
| b | Mit aktiven Substanzen beladener Umgebungsluftstrom |
| b' | Komprimierter, mit aktiven Substanzen beladener Umgebungsluftstrom |

## Patentansprüche

1. Vorrichtung zum Behandeln von Umgebungsluft mit Aktivstoffen, wobei die Vorrichtung ein Gehäuse umfasst, das einen ersten Abschnitt (1) und einen zweiten Abschnitt (1') hat, wobei innerhalb des ersten Abschnitts Aktivstoffträgerelemente (2) angeordnet sind und wobei
- an dem ersten Abschnitt (1) des Gehäuses eine Vorrichtung zum Befördern von Luft (3) von außerhalb des Gehäuses in den ersten Abschnitt (1) des Gehäuses angeordnet ist, und wobei die beförderte Luft die Aktivstoffträgerelemente (2) passiert,
- innerhalb des zweiten Abschnitts des Gehäuses (1') ein Kompressor (5) angeordnet ist, dem Luft aus dem ersten Abschnitt (1) zugeführt wird und der diese Luft über einen Auslass (14) aus dem Gehäuse emittiert,
**dadurch gekennzeichnet, dass**
der Kompressor (5) mit der Vorrichtung zum Befördern von Luft (3) von außerhalb des Gehäuses in den ersten Abschnitt (1) des Gehäuses mittels einer Steuerungs- oder einer Steuerungs-Regelungsvorrichtung (12) operativ derart gekoppelt ist, dass die in das Gehäuse beförderte Luft-Masse (ΔM₁) innerhalb der Zeitspanne ΔT, die einer Anzahl n von Zyklen des Kompressors entspricht, gleich der Luftmasse (ΔM₂) ist, die durch den Auslass (14) innerhalb dieser Zeitspanne ΔT emittiert wird.

2. Vorrichtung nach Anspruch 1, **dadurch gekennzeichnet, dass** die operative Kopplung des Kompressors (5) mit der Vorrichtung zum Befördern von Luft (3) mittels der Steuerungs- oder der Steuerungs-Regelungsvorrichtung (12) eine Synchronisierung einer Antriebsvorrichtung des Kompressors und einer Antriebsvorrichtung der Vorrichtung zum Befördern von Luft (3) ist.

3. Vorrichtung nach Anspruch 1 oder 2, **dadurch gekennzeichnet, dass** die Steuerungs- oder die Steuerungs-Regelungsvorrichtung (12) zumindest eine der Vorrichtungen
- eine Zeitgebervorrichtung, insbesondere eine Zeitschaltuhr (6),
- eine Taktgebervorrichtung,
- eine Vorrichtung zur kontinuierlichen oder stufenweisen Veränderung einer Leistung des Kompressors und/oder der Vorrichtung zum Befördern von Luft (3),
- eine Anzeigevorrichtung,
- ein Bedienfeld,
umfasst.

4. Vorrichtung nach einem der Ansprüche 1 bis 3, **dadurch gekennzeichnet, dass** die Steuerungs- oder die Steuerungs-Regelungsvorrichtung (12) von einem Sensor zur Detektion von Rauch, Temperatur, Luftfeuchtigkeit steuerbar ist, wobei der Sensor geeignet ist, Rauch oder Feuchtigkeit in der Umgebungsluft, die in den ersten Abschnitt (1) des Gehäuses zugeführt wird, oder die Temperatur in dem Gehäuse und der in der Umgebung um das Gehäuse zu messen.

5. Vorrichtung nach einem der vorstehenden Ansprüche, **dadurch gekennzeichnet, dass** das Gehäuse an zumindest einer Seite eine Öffnung aufweist, die durch eine drehgelenkig angelenkte Tür, eine Schiebetür, eine Klappe oder eine Frontplatte eines Einschubelements verschließbar ist.

6. Vorrichtung nach Anspruch 5, **dadurch gekennzeichnet, dass** die Steuerungs- oder die Steuerungs-Regelungsvorrichtung (12) an einer Seite des Gehäuses, insbesondere an der drehgelenkig angelenkten Tür, der Schiebetür, der Klappe oder der Frontplatte des Einschubelements derart angeordnet ist, dass die Steuerungs- oder die Steuerungs-Regelungsvorrichtung (12) von außerhalb des Gehäuses und/oder von innen betätigbar ist.

7. Vorrichtung nach Anspruch 4, **dadurch gekennzeichnet, dass** die operative Kopplung der Steuerungs- oder der Steuerungs-Regelungsvorrichtung (12) mit dem Kompressor (5), mit der Vorrichtung zum Befördern von Luft (3) und mit dem Sensor durch ein Signalübertragungsmittel aus der Gruppe umfassend:
- eine elektronische Steckverbindung (11,13),
- eine berührungslose elektromagnetische Signalübertragungsvorrichtung, insbesondere eine optische oder mittels Funkwellen bereitgestellte Signalübertragungsvorrichtung,
- eine kapazitive Signalübertragungsvorrichtung oder
- eine induktive Signalübertragungsvorrichtung
bereitgestellt wird.

8. Vorrichtung nach einem der Ansprüche 1 bis 7, **dadurch gekennzeichnet, dass** die Vorrichtung an ihrem Auslass (14) mit einem Verteilersystem in Verbindung steht.

9. Vorrichtung nach einem der Ansprüche 1 bis 8, **dadurch gekennzeichnet, dass** die Vorrichtung zum Befördern von Luft (3) von außerhalb des Gehäuses in den ersten Abschnitt (1) des Gehäuses ein Ventilator oder eine Pumpe, insbesondere eine Flügelradpumpe oder eine Flügelzellenpumpe ist.

10. Vorrichtung nach einem der vorstehenden Ansprüche, **dadurch gekennzeichnet, dass** der erste Abschnitt (1) des Gehäuses über dem zweiten Abschnitt des Gehäuses (1') oder neben dem zweiten Abschnitt des Gehäuses (1') angeordnet ist.

11. Vorrichtung nach einem der vorstehenden Ansprüche, **dadurch gekennzeichnet, dass** stromabwärts des Kompressor (5) und stromaufwärts eines Verteilersystems einer oder mehrere Zwischenbehälter, insbesondere Druckbehälter, die einem Druck von mindestens 1 bar, bevorzugt von 10 bar Stand halten, angeordnet sind.

12. Vorrichtung nach Anspruch 11, **dadurch gekennzeichnet, dass** die Zwischenbehälter alternierend oder gleichzeitig dem Verteilersystem zuschaltbar sind.

13. Verfahren zum Behandeln von Umgebungsluft mit Aktivstoffen unter Verwendung einer Vorrichtung nach einem der Ansprüche 1 bis 12, umfassend die Schritte:
- Befördern von Luft mit der Vorrichtung zum Befördern von Luft (3) von außerhalb des Gehäuses in den ersten Abschnitt (1) des Gehäuses,
- Vorbeiführen der Luft an den Aktivstoffträgerelementen und Anreichern der Luft mit Aktivstoffen,
- Zuführen der mit Aktivstoffen angereicherten Luft in einen Kompressor (5),
- Komprimieren der mit Aktivstoffen angereicherten Luft und
- Emittieren der komprimierten, mit Aktivstoffen angereicherten Luft einen Auslass (14) aus dem Gehäuse,
wobei eine Luft-Masse (ΔM₁) innerhalb der Zeitspanne ΔT, die einer Anzahl n von Zyklen des Kompressors entspricht, in das Gehäuse befördert wird, die gleich der Luftmasse (ΔM₂) ist, die durch den Auslass (14) innerhalb dieser Zeitspanne ΔT emittiert wird, indem der Kompressor (5) mit der Vorrichtung zum Befördern von Luft (3) von außerhalb des Gehäuses in den ersten Abschnitt (1) des Gehäuses mittels einer Steuerungs- oder einer Steuerungs-Regelungs-Vorrichtung (12) operativ gekoppelt wird.
